# EUROPÄISCHE PATENTSCHRIFT

(11) **EP 1 552 888 B1**
(45) Veröffentlichungstag und Bekanntmachung des Hinweises auf die Patenterteilung: **20.01.2010**
(21) Anmeldenummer: 04030533.6
(22) Anmeldetag: 22.12.2004
(51) Int. Cl.: B01L 7/00, C12M 1/00

(54) **Klimagerät mit keimdicht abgetrennten Bereichen**
Incubator with germ-tight isolated areas
Incubateur avec des zones isolées étanches aux germes

(30) Priorität: 06.01.2004 DE 102004001104
(43) Veröffentlichungstag der Anmeldung: 13.07.2005
(73) Patentinhaber: Thermo Electron LED GmbH, 63505 Langenselbold (DE)
(72) Erfinder: Stahl, Hermann, 61130 Nidderau-Ostheim (DE); Brömsen, Olaf, 64546 Mörfelden (DE); Pieczarek, Waldemar, 63486 Bruchköbel (DE); Hohenthanner, Ulrike, Dr., 63457 Hanau (DE); Schuck, Rainer, 63775 Alzenau (DE)
(74) Vertreter: Tomerius, Isabel

(56) Entgegenhaltungen:
- US-A- 4 336 329
- US-A- 4 622 049
- US-A- 5 858 770
- US-A- 5 890 703
- US-A- 5 908 776

## Beschreibung

Die Erfindung betrifft ein Klimagerät mit einem Innenraum, welcher einen Nutzraum umfasst, und mit einer verschließbaren Zugriffsöffnung zum Nutzraum, wobei mindestens ein Teil des Innenraumes vom Rest des Innenraums unter Bildung eines keimdicht abgetrennten Raumes abgetrennt und keimdicht abgedichtet ist und dass die Abtrennung zumindest teilweise durch eine Membran erfolgt, die durchlässig ist für Wasserdampf und Gas, aber undurchlässig für Mikroorganismen, und wobei es mindestens ein Membranbehältnis aufweist, welches mindestens einen Lagerplatz für Proben mit Außenwänden keimdicht umschließt, wobei mindestens ein Teilbereich dieser Außenwände von einer Membran gebildet wird, welche durchlässig ist für Wasserdampf und Gas, aber undurchlässig für Mikroorganismen, sowie ein Membranbehältnis zur Verwendung in einem solchen Klimagerät.

Ein Klimagerät ist ein Laborgerät zur Aufbewahrung von vorwiegend biologischen oder chemischen Proben unter bestimmten klimatischen Bedingungen. Beispiele hierfür sind Inkubatoren und Kühl- oder Gefriergeräte. Solche Klimageräte haben einen Nutzraum der von einem Gehäuse gasdicht umschlossen und thermisch isoliert wird. Der Nutzraum ist über eine Öffnung, welche häufig die gesamte Vorderseite des Klimagerätes einnimmt und die durch eine Verschlussvorrichtung gasdicht abgeschlossen werden kann, zugänglich. Für die Lagerung von Proben ist der Nutzraum in der Regel regalartig mit horizontalen Einlagen versehen. Derartige Klimageräte bieten in ihrem Nutzraum ein Klima mit vorgegebener Temperatur und Luftfeuchtigkeit und im Falle von Inkubatoren auch eine vorgegebene CO₂-Konzentration. Die Verschlussvorrichtung ist häufig eine äußere Schwenktür, hinter welcher üblicherweise eine transparente innere Tür als Gasblende angeordnet ist. Diese verhindert, dass beim Öffnen der äußeren Schwenktür ein zu schneller Gasaustausch zwischen Nutzraum und Umgebung stattfindet. Gleichzeitig erlaubt die transparente Gasblende aber den Blick auf die Proben. Öffnungszeiten der Gasblende können so auf ein Minimum reduziert und damit unnötige Störungen des Nutzraumklimas weitgehend vermieden werden. Um Störungen noch weiter einzuschränken, sind geteilte Einlagen und unterteilte Gasblenden bekannt. Beispielsweise gibt es Gasblenden mit sechs separaten Zugriffsöffnungen, die jeweils über eine eigene Tür verschließbar sind. Solche unterteilten Gasblenden ermöglichen es, gezielt auf bestimmte Einlagen und Lagerplätze zurückzugreifen, wobei die anderen Lagerplätze geschützt bleiben.

Eine gängige Methode zur Befeuchtung der Atmosphäre eines Inkubators erfolgt über passive Verdunstung von Wasser in einem offenen Wasserbad. Dem Vorteil einer sehr homogenen Feuchtigkeitsverteilung in der Luft steht der Nachteil der offenen Wasseroberfläche entgegen. Schmutzpartikel, Viren, Pilze, Bakterien und Mykoplasmen reichern sich in dem Wasserbad in großer Zahl an, wenn sie durch Hineintropfen oder Staubeintrag durch die Luft erst einmal in das Wasser gelangt sind.

Eine Kontaminationsgefahr besteht für die Proben auch durch andere Proben. Einerseits können die Proben von fremden Keimen wie Pilzen oder Bakterien befallen werden. Andererseits kann es sich bei den Proben selbst um gefährliche Erreger handeln. Dies kann eine Gefahr sowohl für andere Proben durch Kreuzkontamination als auch für Bedienpersonen bedeuten. Wenn der Nutzraum des Klimagerätes einmal kontaminiert ist, ist meist eine aufwändige Desinfektion des gesamten Gerätes erforderlich, was lange Ausfallzeiten zur Folge hat.

Aus der US 5858770 ist es bekannt, ein Probengefäß, wie eine Mikrotiterplatte oder eine Multiwellplatte, durch eine keimdichte, aber wasserdampf- und gasdurchlässige Membran abzudecken. Die sterile Membran wird dabei mittels einer Klebstoffschicht auf der Mikrotiterplatte befestigt und ist vor ihrem einmaligen Gebrauch durch eine Folie ihrerseits vor Kontamination geschützt. Die Membran bildet so einen einmalig verwendbaren keimdichten Verschluss für die Probengefäße.

Zur Verhinderung und Vorbeugung von Kontamination und Kreuzkontamination sind auch Membranbeutel aus keimdichter Membran bekannt. Einzelne Probenträger können in solche Membranbeutel verpackt und verschlossen in einem Klimagerät gelagert werden. In ihrer Handhabung werden derartige Membranbeutel jedoch als unkomfortabel angesehen. Dementsprechend finden sie eine geringe Akzeptanz bei den Benutzern.

Vor diesem Hintergrund ist es Aufgabe der Erfindung ein Klimagerät anzugeben, welches die Gefahr der Kontamination des gesamten Gerätes und der Kreuzkontamination vermindert und trotzdem bedienerfreundlich ist.

Die Aufgabe wird gelöst durch ein Klimagerät gemäß dem Anspruch 1 sowie durch ein Membranbehältnis gemäß Anspruch 12. Vorteilhafte Weiterbildungen sind in den abhängigen Ansprüchen 2 bis 11 und 13 bis 28 aufgeführt.

Ein Klimagerät nach Anspruch 1 hat demnach einen Innenraum, welcher einen Nutzraum umfasst, und eine verschließbare Zugriffsöffnung zum Nutzraum, wobei mindestens ein Teil des Innenraums vom Rest des Innenraums unter Bildung eines keimdicht abgetrennten Raumes abgetrennt und keimdicht abgedichtet ist und die Abtrennung zumindest teilweise durch einen Membran erfolgt, die durchlässig ist für Wasserdampf und Gas, aber undurchlässig für Mikroorganismen.

Der Vorteil gegenüber dem Stand der Technik liegt darin, dass eine auftretende Kontamination in einem Teil des Innenraumes nicht auf die anderen Teile des Innenraumes übergreifen kann. Eine Kreuzkontamination zwischen verschiedenen keimdicht abgetrennten Teilen ist nicht möglich. Daher muss im Falle einer Kontamination nicht mehr der gesamte Innenraum desinfiziert werden, sondern nur ein Teilbereich.

Die keimdichte Abtrennung erfolgt zumindest teilweise über eine Membran, die durchlässig ist für Wasserdampf und Gas, aber undurchlässig für Mikroorganismen. Die Durchlässigkeit der Membran wird durch ihre Poren bestimmt. Die Poren müssen ausreichend groß sein, damit Wasserdampf und Gas die Membran möglichst ungehindert durchdringen können. Andererseits sollte die Porengröße derart nach oben beschränkt werden, dass auch kleinste Mikroorganismen die Poren nicht durchdringen können. Vorzugsweise weist die Membran daher Poren auf, deren Größe maximal 0,5 µm und besonders bevorzugt kleiner als 0,2 Mikrometer ist.

Das Klimagerät kann ein Gerät sein, das lediglich die Temperatur des Innenraums regelt, wie ein Kühl- oder Gefriergerät. Vorzugsweise ist das Klimagerät ein Gerät, das zusätzlich die Einstellung der Nutzraum-Atmosphäre erlaubt, wie ein Inkubator.

In einer bevorzugten Ausführungsvariante hat das Klimagerät zwischen der Zugriffsöffnung und dem Inneren des Klimagerätes eine Gasblende, die den Innenraum des Klimagerätes abschließt. Sowohl eine Ausführung der Gasblende als Schwenktür als auch eine auf Schienen herausziehbare Gasblende können vorteilhaft sein. Besonders bevorzugt ist eine unterteilte Gasblende mit mehreren Öffnungen. Mehrere Öffnungen gestatten den Zugriff auf einzelne Fächer im Klimagerät, während die Atmosphäre in den übrigen Fächern nur wenig gestört wird.

Durch keimdichte Abtrennung einzelner Räume im Innenraum des Klimageräts mittels einer Membran werden Störungen der Atmosphäre noch weiter verringert. Eine solche Membran kann beispielsweise über einen Rahmen gespannt und an diesem befestigt sein.

Günstig ist es auch, wenn elektrische Komponenten wie Motoren oder Lüfter und Sensoren zum Schutz vor Kontamination in einem solchen keimdicht abgetrennten Raum untergebracht sind. Auch ein Wasserbad zur Befeuchtung des Nutzraums kann auf diese Weise durch eine Membran geschützt werden. Da die Membran für Gas und Wasserdampf durchlässig ist, kann Feuchtigkeit in den gesamten Nutzraum diffundieren. Besonders gefördert wird dies, wenn das Wasserbad temperiert ist und wenn zusätzlich ein Ventilator in dem abgetrennten Raum ist, der die Diffusion der feuchten Luft begünstigt. Eine Möglichkeit besteht auch darin, feuchte Luft mittels eines Ventilators durch einen Membranschlauch zu spülen, der durch den Nutzraum geführt wird und so für eine gleichmäßige Feuchtigkeit in diesem sorgt.

Dabei kann durch Ausgasen der Proben oder ähnliches die Feuchtigkeit im Nutzraum zu hoch ansteigen. Deshalb kann eine Wärmebrücke (cold spot) zwischen dem abgetrennten Raum mit Wasserbad und dem Nutzraum angebracht werden. Überschüssige Feuchtigkeit im Nutzraum kondensiert bevorzugt daran und kann zurück in das Wasserreservoir geleitet werden.

Erfindungsgemäß wird ein Teil des Nutzraums durch ein Membranbehältnis keimdicht abgetrennt. Das Membranbehältnis ist von Wänden keimdicht umschlossen, wobei mindestens ein Teil der Wandflächen aus Membran besteht. Die Membran sollte so bemessen sein, dass ein ausreichender Gas- und Feuchtigkeitsaustausch stattfinden kann. Im Allgemeinen ist es ausreichend, wenn beispielsweise nur eine Seitenwand aus Membran gefertigt ist und der Rest der Wände aus gasdichtem Material besteht. Ein Membranbehältnis bietet dabei Platz für ein oder mehrere Probengefäße.

Das Membranbehältnis hat erfindungsgemäß mindestens eine Öffnung zum Be- und Entladen mit Proben, die mit einer Verschlussvorrichtung lösbar keimdicht verschließbar ist. Vorzugsweise besteht diese Verschlussvorrichtung aus einer Tür. Andere Varianten sind Membranteile, die übereinandergeschlagen werden, oder auch ein Schraubverschluss. Sinnvoll ist es, wenn das Membranbehältnis je eine solche Ladeöffnung an der Vorderseite und an der Rückseite hat. Das Membranbehältnis kann dann mit jeder der Seiten voran in das Klimagerät eingeschoben werden.

Das Klimagerät wiederum kann entweder ein oder auch mehrere Membranbehältnisse aufweisen. Im Extremfall nimmt ein Membranbehältnis den gesamten Nutzraum des Klimagerätes ein. Wesentlich bei mehreren Membranbehältnissen ist, dass sie nicht gegenseitig die Membranen verdecken, so dass die Diffusion von Gas und Feuchtigkeit nicht behindert wird. Es kann daher sinnvoll sein, nicht den gesamten Nutzraum des Klimagerätes mit Membranbehältnissen auszufüllen, sondern Zwischenräume frei zu lassen, damit die Diffusion ungehindert stattfinden kann. Um die Diffusion zusätzlich zu verbessern, kann der Luftwechsel im Nutzraum erhöht werden, zum Beispiel durch Anordnen von Ventilatoren.

Besonders bevorzugt ist der ganze Nutzraum des Klimagerätes durch Membranbehältnisse unterteilt. Eine Kreuzkontamination zwischen den einzelnen Behältnissen ist nicht möglich. Falls Kontamination in einem Membranbehältnis auftritt, kann das betroffene Behältnis ersetzt und einzeln desinfiziert oder vernichtet werden. Ein weiterer Vorteil liegt darin, dass die Luftfeuchtigkeit in dem erfindungsgemäßen Klimagerät gegenüber dem Stand der Technik erhöht werden kann. Die Gefahr von Kontamination durch Kondenswasser wird durch die Membranabtrennungen stark vermindert. Eine höhere Feuchtigkeit bietet einen besseren Schutz vor Austrocknung der Proben.

Die einzelnen Membranbehältnisse können fest im Klimagerät montiert sein oder vorzugsweise mit einer Befestigungsvorrichtung lösbar im Innenraum befestigbar sein. Im Innenraum des Klimagerätes kann zum Beispiel ein Gestell oder ein Rahmen vorhanden sein, in den mehrere Membranbehältnisse eingeschoben werden. Auch ein Ausziehmechanismus in der Art von Schubladen ist möglich. Besonders günstig und materialsparend kann eine Aufhängung der Membranbehältnisse im Innenraum sein. Zweckmäßig ist es auch, wenn das Membranbehältnis an einer oder mehreren Innenwandungen des Klimageräts befestigt ist.

In einer Ausführungsvariante ist das Membranbehältnis an der Gasblende des Klimagerätes lösbar befestigt. Dabei kann es einerseits von vorne in eine Öffnung der verschlossenen Gasblende eingesteckt und mit einem Schnellverschluss befestigt werden. In einer anderen Variante ist das Membranbehältnis fest an der Rückseite der Gasblende montiert. Dann kann das Membranbehältnis beim Öffnen der Gasblende mit aus dem Innenraum des Klimagerätes geklappt oder gezogen werden. In diesem Fall dichtet das Membranbehältnis zweckmäßig an der Vorderseite über eine Dichtung gegen die Gasblende ab. Die Gasblende bildet dann die Vorderwand des Mem branbehältnisses.

Wenn das Membranbehältnis an der Vorderseite gegen die Gasblende abdichtet, ist es besonders vorteilhaft, wenn eine Verschlussvorrichtung in die Gasblende integriert ist. Dabei kann das Membranbehältnis entweder im Innenraum des Klimagerätes befestigt sein, zum Beispiel an einem Rahmen oder einem Gestell, und an seiner offenen Vorderseite über eine flexible Dichtung gegen die Gasblende abdichten. Zur Erhöhung des Anpressdruckes sind Federungssysteme vorteilhaft. Der Innenraum des Membranbehältnisses ist in dieser Ausführungsform über eine in die Gasblende integrierte Tür erreichbar. Die Tür ist dann zweckmäßig in ihrem Querschnitt kleiner als der Querschnitt des Membranbehältnisses, mit dem dieses an der Gasblende abdichtet. Der Vorteil einer solchen Ausführung liegt in der einfachen Bedienbarkeit. Jedes Membranbehältnis ist direkt über eine Tür in der Gasblende erreichbar.

In einer weiteren bevorzugten Variante ist die Verschlussvorrichtung direkt am Membranbehältnis befestigt. Dies ermöglicht, dass das gesamte Membranbehältnis im keimdicht verschlossenen Zustand aus dem Klimagerät herausgezogen werden kann. Dadurch können Proben in keimdichter Umgebung von einem Gerät zu anderen Geräten, beispielsweise in Vakuumschränke, Trockenschränke oder mikrobiologische Sicherheitswerkbänke, transportiert werden. Ein solches Membranbehältnis wird zweckmäßigerweise von vorne in eine Gasblende geschoben. Eine Gasblende muss dafür Öffnungen haben, die dem Querschnitt der Membranbehältnisse angepasst sind. Wenn ein Membranbehältnis aus der Gasblende herausgezogen wurde, kann der entstandene Durchlass mit einer Ersatzverschlussvorrichtung abgedeckt werden. Dies ist auch durch einen automatischen Mechanismus möglich.

Die Aufgabe der Erfindung wird auch durch ein Membranbehältnis zur Verwendung in einem Klimagerät gelöst, wobei das Membranbehältnis mindestens einen Probenlagerplatz mit Wänden keimdicht umschließt und zumindest ein Teilbereich dieser Wände von einer Membran gebildet wird. Die Membran ist durchlässig für Gas und Wasserdampf, aber undurchlässig für Mikroorganismen. Das Membranbehältnis weist mindestens eine Ladeöffnung auf, die durch eine Verschlussvorrichtung, insbesondere durch eine Tür, lösbar keimdicht verschließbar ist. Wie oben beschrieben, kann die Verschlussvorrichtung auch aus Membranteilen oder einem Schraubverschluss bestehen. Zweckmäßig ist es, wenn die Ladeöffnung mit der Zugriffsöffnung des Klimageräts ausgerichtet ist. Die Verschlussvorrichtung kann auch funktionell mit der Zugriffsöffnung des Klimageräts verbunden sein.

Es ist meist ausreichend, wenn das Membranbehältnis nur ein Membranfenster aufweist und ansonsten gasdicht gestaltet ist. Zum gasdichten Verschließen des Membranbehältnisses reicht es dann, wenn das Membranfenster gasdicht abgedeckt wird. Das Membranbehältnis kann auch abschließbar gestaltet sein.

Vorzugsweise hat die Membran Poren mit einer Größe von maximal 0,5 µm und besonders bevorzugt von kleiner als 0,2 µm.

[0026] In einer bevorzugten Weiterbildung weist das Membranbehältnis mindestens ein Sichtfenster auf. Ein Sichtfenster ist sinnvoll an der Vorderseite, insbesondere wenn das Membranbehältnis mit seiner Vorderseite in die Gasblende integriert ist. Zusätzlich kann ein Sichtfenster in der Oberseite sinnvoll sein, damit man die Proben gut beobachten kann, wenn das Membranbehältnis aus dem Klimagerät herausgenommen ist. Ein Sichtfenster auf der Unterseite ist sinnvoll, wenn die Proben mit einem Inversmikroskop untersucht werden sollen, ohne aus der keimdichten Atmosphäre entnommen zu werden. Ein aus dem Klimagerät herausgenommenes Membranbehältnis ist zweckmäßig selbsttragend und mit anderen gleichartigen Membranbehältnissen stapelbar. Vorteilhaft ist es, wenn das Membranbehältnis in seiner Form den zu verwendenden Probengefäßen angepasst ist. In der Regel ist es quaderförmig. Für die keimdichte Lagerung von Flaschen können dagegen zylindrische Membranbehältnisse sinnvoller sein. Ein zylindrisches Membranbehältnis kann auch anstelle einer Tür zweckmäßigerweise mit einem Schraubverschluss versehen sein. Zweckmäßig ist auch eine Anpassung der Membranbehältnisse an die Form des Klimageräts, so dass die Membranbehältnisse passgenau in das Klimagerät ein- und ausgeführt werden können.

[0027] In einer weiteren Ausführungsvariante hat das Membranbehältnis eine Verschlussvorrichtung, die beim Herausnehmen des Membranbehältnisses aus dem Klimagerät selbsttätig schließt. Dies kann beispielsweise eine Klappe sein, die sich beim Einstecken in ein Klimagerät öffnet und beim Herausnehmen automatisch zurückfällt. Für den Transport von unbefüllten Membranbehältnissen ist es zweckmäßig, wenn diese, ähnlich einer Papiertüte oder einer Ziehharmonika, flach zusammengefaltet werden können.

Ein Membranbehältnis ist zweckmäßigerweise aus sterilisierbarem Material gefertigt. Dies kann bei Dampfsterilisation zum Beispiel hitzebeständiger Kunststoff sein. Nach einer Kontamination sind auch chemische Methoden zur Desinfektion möglich. Im Falle einer Kontamination mit gefährlichen Erregern ist auch eine Sterilisation in einem Gerät denkbar, bei der das Membranbehältnis zerstört wird. Bei ihrer Auslieferung im Verkauf sind unbenutzte Membranbehältnisse vorzugsweise selbst steril, steril verpackt und platzsparend gefaltet.

Für den Transport eines gefüllten Membranbehältnisses hat es vorzugsweise einen Transportbehälter, in den das Membranbehältnis wasserdampf- und gasdicht einschließbar ist. Ein solcher Transportbehälter verfügt vorzugsweise über wenigstens ein Sichtfenster oder ist zumindest teilweise oder ganz aus transparentem Material gefertigt.

In einer vorteilhaften Weiterbildung hat der Transportbehälter eine Vorrichtung zur Temperierung für längere Transporte. Dies kann durch batteriegepufferte Systeme erfolgen oder auch einfach durch ein wärmespeicherndes Material mit hoher Wärmekapazität. Eine einfachere Variante hält die Temperatur allein durch thermische Isolierung.

Auch eine Vorrichtung zum Erhalt und zur Steuerung der Gaszusammensetzung in dem Transportbehälter ist zweckmäßig. Dies geschieht zum Beispiel durch eine angeschlossene transportable Gasflasche.

Für den Erhalt der Luftfeuchtigkeit im Transportbehälter weist dieser vorzugsweise eine Befeuchtungsvorrichtung auf. Dies kann ein Wasserreservoir in dem Transportbehälter sein. Um das Fließen von Wasser im Transportbehälter zu vermeiden, wird vorzugsweise eine mit Wasser getränkte Schwammstruktur als Befeuchtungsvorrichtung verwendet. Für Heizung, Gaszufuhr und aktive Befeuchtung sind batteriebetriebene Systeme möglich, die über eigene Regelkreise steuerbar sein können.

Zusätzlich kann der Transportbehälter abschließbar gestaltet sein.

In einer bevorzugten Weiterbildung weisen das Membranbehältnis oder der Transportbehälter ein Lesegerät zur Ermittlung von Informationen über Proben auf, welches mit einer Auswertungseinheit kommunizieren kann. Die Informationen über Proben können auf Barcode oder Magnetstreifen gespeichert sein. Beim Einbringen von Proben in das Membranbehältnis oder in den Transportbehälter werden diese Informationen von einem Lesegerät automatisch erfasst. Das Lesegerät wiederum kann vorzugsweise mit einer Auswertungseinheit kommunizieren. Für Kontrollzwecke und Datenerfassung wird es beispielsweise an einen PC angeschlossen. Diese Kommunikation kann vorzugsweise auch drahtlos zum Beispiel mit Bluetooth-Technik erfolgen.

Zum Befördern eines Membranbehältnisses aus einem Klimagerät in einen Transportbehälter bei möglichst geringer Störung der Atmosphäre im Membranbehältnis hat der Transportbehälter in einer bevorzugten Ausführungsform eine Öffnung, welche gegen die Gasblende eines Klimagerätes gasdicht abdichtbar ist. Er weist außerdem ein Abschlussmittel auf, mit welchem das Eindringen von Außenatmosphäre in den Transportbehälter im Wesentlichen verhindert wird. Der Transportbehälter kann zweckmäßig so an die Gasblende des Klimageräts angebracht werden, dass die Öffnung des Transportbehälters die Frontseite des in der Gasblende steckenden Membranbehältnisses umschließt. Das Membranbehältnis wird dann in den Transportbehälter gezogen. Während dieses Übergangs kann das Membranbehältnis stets in kontrollierter Atmosphäre bleiben. Der umgekehrte Transportweg ist genauso möglich. Dabei kann das Membranbehältnis in einer vorteilhaften Ausführung so gestaltet sein, dass die Rückwand des Membranbehältnisses selbst gasdicht ist und im Transportbehälter dessen gasdichte Rückwand bildet.

In einer anderen Variante wird das Membranbehältnis vollständig in den Transportbehälter eingeschoben, und hinter dem Membranbehältnis wird der Transportbehälter mit einer Schiebevorrichtung vorschlossen, bevor der Transportbehälter von der Gasblende gelöst wird.

In folgenden soll die Erfindung anhand der in den Figuren dargestellten Ausführungsbeispiele weiter erläutert werden.

Es zeigen schematisch:
- Fig. 1: eine Seitenansicht eines Klimagerätes im vertikalen Schnitt durch die vordere Mitte mit durch Membranen abgetrennten Räumen;
- Fig. 2: die geöffnete Vorderseite eines Klimagerätes in Frontalansicht mit weggeklappter Gasblende und nach vorne offenen Membranbehältnissen und separaten, in die Gasblende integrierten Türen;
- Fig. 3: eine Ansicht von oben auf ein nach vorne offenes Membranbehältnis, welches gegen die Gasblende abdichtet und dessen Tür an der Gasblende angebracht ist;
- Fig. 4: eine Ansicht von oben auf eine andere Version eines Membranbehältnisses mit daran angebrachter Tür, welche von vorne in die Gasblende eingeschoben ist;
- Fig. 5: eine Seitenansicht eines herausnehmbaren Membranbehältnisses nach Fig. 4 im Schnitt, welches in einen Transportbehälter eingeschoben wird und dessen gasdichte Rückwand die Rückwand des Transportbehälters bildet, und
- Fig. 6: eine Seitenansicht im Schnitt einer anderen Version eines Membranbehältnisses nach Fig. 4 in einem Transportbehälter, welcher durch eine Schiebevorrichtung hinter dem Membranbehältnis verschlossen wird.

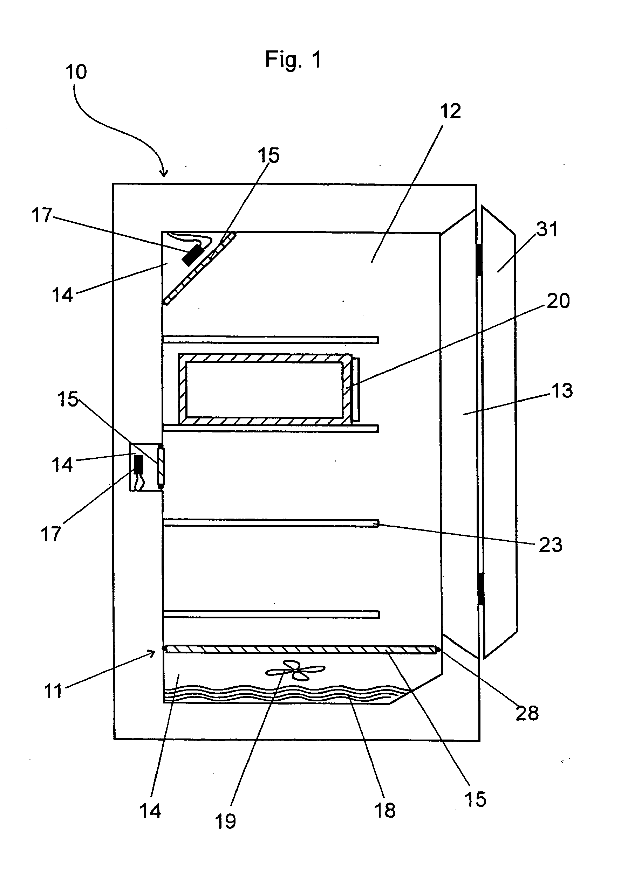

Fig. 1 zeigt ein Klimagerät 10 mit einem Innenraum 11, der mit einer verschließbaren Zugriffsöffnung 13 erreichbar ist. Die Schwenktür 31, welche den Innenraum 11 verschließt, ist aufgeklappt in Seitenansicht dargestellt. Im Innenraum des Klimagerätes befinden sich mehrere durch Membranen 15 abgeteilte Räume 14. Der Boden des Innenraumes ist von einem Wasserbad 18 bedeckt. Das Wasserbad ist durch eine auf einen Rahmen aufgespannte Membran 15 und Dichtungen 28 entlang der Seitenwände des Innraums gegenüber dem Nutzraum 12 abgedichtet. Zur besseren Diffusion im Innenraum 11 ist der Raum 14 mit dem Wasserbad zusätzlich mit einem Ventilator 19 ausgestattet. In der Rückwand des Innenraums befindet sich eine Ausnehmung, welche durch eine Membran 15 gegenüber dem Nutzraum 12 abgedichtet ist. Im Inneren dieses abgetrennten Raumes 14 befindet sich ein Sensor 17. Ein weiterer Sensor 17 ist in einem durch eine Membran 15 abgetrennten Raum 14, im Winkel zwischen Decke und Rückwand des Innenraums angebracht. Für die Lagerung von Proben ist der Innenraum regalartig mit horizontalen Einlagen 23 versehen. Auf einer Einlage befindet sich ein verschlossenes Membranbehältnis 20, welches einen zusätzlichen Teil des Innenraums durch eine Membran abtrennt.

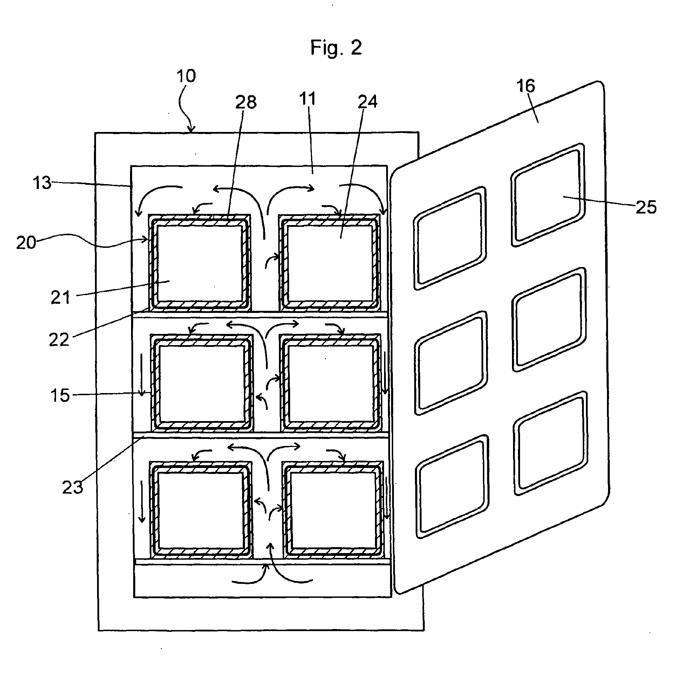

In Fig. 2 sind sechs solcher Membranbehältnisse 20 im Innenraum 11 eines Klimagerätes dargestellt. Diese Ausführungsvariante von Membranbehältnissen 20 ist nach vorne offen und mit einer Dichtung 28 ausgestattet. Die Membranbehältnisse werden durch eine Gasblende 16, welche hier im weggeklappten Zustand dargestellt ist, nach vorne abgedichtet. Jedes Membranbehältnis ist durch eine separate Tür 25 in der Gasblende 16 zugänglich. Die Membranbehältnisse 20 füllen nicht den gesamten Innenraum des Klimagerätes 10 aus, so dass Diffusion von feuchter Atmosphäre stattfinden kann. Die Pfeile in der Darstellung illustrieren die Diffusion, sollen aber keinerlei Einschränkung der tatsächlichen Strömungen im Klimagerät bedeuten.

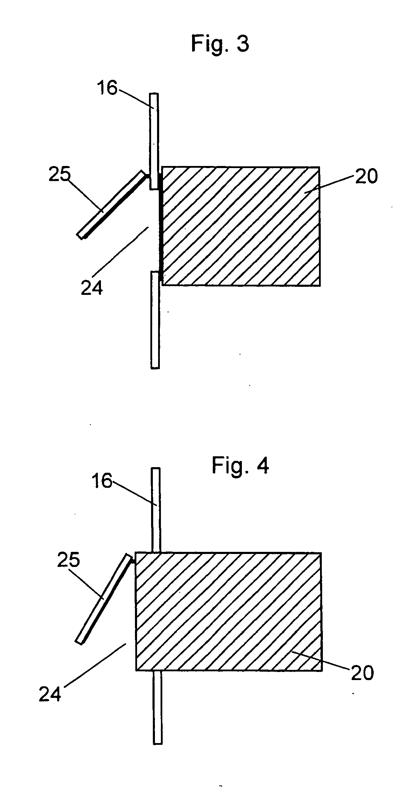

Fig. 3 zeigt ein Membranbehältnis 20, wie in Fig. 2, von oben gesehen. Das Membranbehältnis 20 schließt an seiner Öffnung 24 mit einer Dichtung 28 gegen die Gasblende 16. Die Öffnung in der Gasblende ist durch eine an der Gasblende angebrachte Tür 25 verschließbar.

Eine andere Version eines Membranbehältnisses ist in Fig. 4 dargestellt. Dieses Membranbehältnis 20 hat eine Tür 25, welche an dem Membranbehältnis selber angebracht ist. Diese Ausführungsvariante kann im Ganzen verschlossen aus dem Klimagerät 11 herausgenommen werden. Im Klimagerät 10 eingebaut, steckt sie von vorne in der Öffnung einer Gasblende 16.

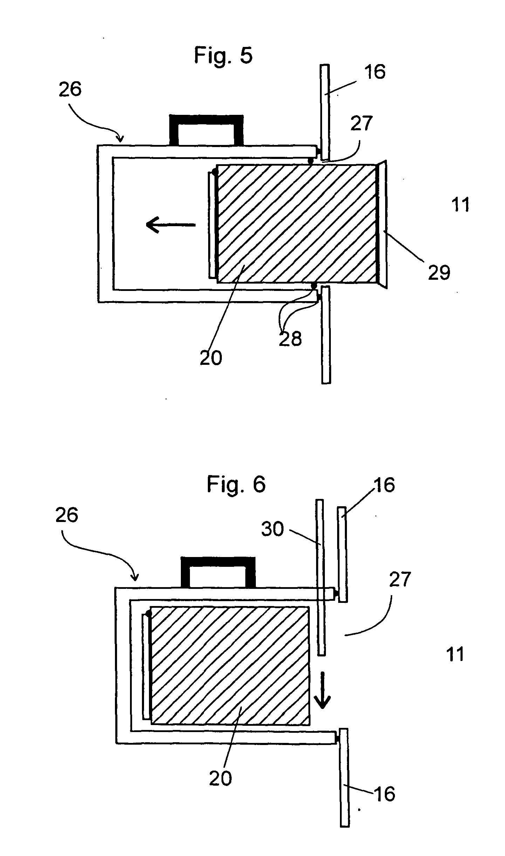

In Fig. 5 ist eine Weiterbildung dieser Ausführungsvariante dargestellt. Das Membranbehältnis 20 steckt zur Hälfte im Innenraum 11 eines Klimagerätes und wird durch eine Gasblende 16 in einen Transportbehälter 26 geschoben. Der Transportbehälter 26 hat einen Tragegriff und ist über Dichtungen 28 an die Gasblende 16 angeschlossen. Das Membranbehältnis 20 hat eine gasdichte Rückwand 29, die im eingeschobenen Zustand auch die Rückwand des Transportbehälters 26 bildet.

Eine andere Variante eines Membranbehälters 20 in einem Transportbehälter 26 zeigt Fig. 6. In dieser Darstellung ist das Membranbehältnis vollständig in den Transportbehälter eingeschoben. Der Transportbehälter 26 ist über Dichtungen gegen die Gasblende 16 des Klimagerätes angeschlossen. Mit einer Schiebevorrichtung 30 wird der Transportbehälter 26 verschlossen, während er noch an die Gasblende 16 und die Atmosphäre des Klimagerätes 10 angeschlossen ist. Erst wenn der Transportbehälter 26 verschlossen ist, wird er von dem Klimagerät gelöst.

## Patentansprüche

1. Klimagerät (10) mit einem Innenraum (11), welcher einen Nutzraum (12) umfasst, und mit einer verschließbaren Zugriffsöffnung (13) zum Nutzraum, wobei mindestens ein Teil des Innenraumes vom Rest des Innenraums unter Bildung eines keimdicht abgetrennten Raumes (14) abgetrennt und keimdicht abgedichtet ist und dass die Abtrennung zumindest teilweise durch eine Membran (15) erfolgt, die durchlässig ist für Wasserdampf und Gas, aber undurchlässig für Mikroorganismen, und wobei es mindestens ein Membranbehältnis (20) aufweist, welches mindestens einen Lagerplatz (21) für Proben mit Außenwänden (22) keimdicht umschließt, wobei mindestens ein Teilbereich dieser Außenwände von einer Membran (15) gebildet wird, welche durchlässig ist für Wasserdampf und Gas, aber undurchlässig für Mikroorganismen,
**dadurch gekennzeichnet,**
**dass** das Membranbehältnis (20) mindestens eine Ladeöffnung (24) aufweist, die mit einer Verschlussvorrichtung (25) lösbar keimdicht verschließbar ist.

2. Klimagerät nach Anspruch 1,
**dadurch gekennzeichnet,**
**dass** die Membran Poren aufweist mit einer Porengröße von maximal 0,5 µm und besonders bevorzugt von kleiner als 0,2 µm.

3. Klimagerät nach Anspruch 1 oder 2,
**dadurch gekennzeichnet,**
**dass** das Klimagerät ein Inkubator ist.

4. Klimagerät nach einem der vorhergehenden Ansprüche,
**dadurch gekennzeichnet,**
**dass** eine Gasblende (16) zwischen der Zugriffsöffnung (13) und dem Inneren (11) des Klimagerätes angeordnet ist, wobei die Gasblende den Innenraum des Klimageräts (10) abschließt.

5. Klimagerät nach einem oder mehreren der Ansprüche 1 bis 4,
**dadurch gekennzeichnet,**
**dass** es eine Befestigungsvorrichtung (23) aufweist, an der das Membranbehältnis (20) im Innenraum (11) des Klimageräts lösbar befestigbar ist.

6. Klimagerät nach Anspruch 5,
**dadurch gekennzeichnet,**
**dass** das Membranbehältnis (20) lösbar an der Gasblende (16) befestigt ist.

7. Klimagerät nach einem der vorherigen Ansprüche,
**dadurch gekennzeichnet,**
**dass** die Verschlussvorrichtung (25) eine Tür ist.

8. Klimagerät nach einem der vorherigen Ansprüche,
**dadurch gekennzeichnet,**
**dass** die Verschlussvorrichtung (25) in die Gasblende (16) integriert ist und das Membranbehältnis (20) gegen die Gasblende (16) abdichtet.

9. Klimagerät nach einem der Ansprüche 1 bis 7,
**dadurch gekennzeichnet,**
**dass** die Verschlussvorrichtung (25) am Membranbehältnis (20) befestigt ist und gegen dieses abdichtet.

10. Klimagerät nach Anspruch 9,
**dadurch gekennzeichnet,**
**dass** das Membranbehältnis (20) zusammen mit der Verschlussvorrichtung (25) aus dem Klimagerät (10) herausnehmbar ist.

11. Klimagerät nach Anspruch 10,
**dadurch gekennzeichnet,**
**dass** es eine Ersatzverschlussvorrichtung aufweist, welche einen durch Herausnehmen eines Membranbehältnisses (20) entstandenen Durchlass verschließt.

12. Membranbehältnis zur Verwendung in einem Klimagerät nach einem der Ansprüche 10 und 11, welches mindestens einen Probenlagerplatz mit Wänden (22) keimdicht umschließt , wobei zumindest ein Teilbereich der Wände von einer Membran (15) gebildet wird, welche durchlässig ist für Gas und Wasserdampf, aber undurchlässig für Mikroorganismen, und welches mindestens eine Ladeöffnung (24) aufweist,
**dadurch gekennzeichnet,**
**dass** die Ladeöffnung durch eine Verschlussvorrichtung (25), insbesondere durch eine Tür, lösbar keimdicht verschließbar ist.

13. Membranbehältnis nach Anspruch 12,
**dadurch gekennzeichnet,**
**dass** die Membran Poren aufweist mit einer Porengröße von maximal 0,5 µm und besonders bevorzugt von kleiner als 0,2 µm.

14. Membranbehältnis nach einem der Ansprüche 12oder 13,
**dadurch gekennzeichnet,**
**dass** es mindestens ein Sichtfenster aufweist.

15. Membranbehältnis nach einem oder mehreren der Ansprüche 12 bis 14,
**dadurch gekennzeichnet,**
**dass** es eine Verschlussvorrichtung aufweist, die es beim Herausnehmen aus dem Klimagerät selbsttätig verschließt.

16. Membranbehältnis nach einem oder mehreren der Ansprüche 12 bis 15,
**dadurch gekennzeichnet,**
**dass** es für den Transport flach zusammenfaltbar ist.

17. Membranbehältnis nach einem oder mehreren der Ansprüche 12 bis 16,
**dadurch gekennzeichnet,**
**dass** es aus sterilisierbarem Material gefertigt ist.

18. Membranbehältnis nach einem oder mehreren der Ansprüche 12 bis 17,
**dadurch gekennzeichnet,**
**dass** es einen Transportbehälter (26) aufweist, in den das Membranbehältnis (20) zum Transport wasserdampf- und gasdicht einschließbar ist.

19. Membranbehältnis nach Anspruch 18,
**dadurch gekennzeichnet,**
**dass** der Transportbehälter (26) wenigstens teilweise aus transparentem Material gefertigt ist.

20. Membranbehältnis nach einem oder mehreren der Ansprüche 18 und 19,
**dadurch gekennzeichnet,**
**dass** der Transportbehälter (26) eine Vorrichtung zur Temperierung aufweist.

21. Membranbehältnis nach einem oder mehreren der Ansprüche 18 bis 20,
**dadurch gekennzeichnet,**
**dass** der Transportbehälter (26) eine Vorrichtung aufweist, mit der die Gaszusammensetzung in seinem Inneren beeinflusst werden kann.

22. Membranbehältnis nach einem oder mehreren der Ansprüche 18 bis 21,
**dadurch gekennzeichnet,**
**dass** der Transportbehälter (26) eine Befeuchtungsvorrichtung aufweist, mit der die Luftfeuchtigkeit im Inneren reguliert werden kann.

23. Membranbehältnis nach Anspruch 22,
**dadurch gekennzeichnet,**
**dass** die Befeuchtungsvorrichtung mindestens eine mit Wasser getränkte Schwammstruktur aufweist.

24. Membranbehältnis nach einem oder mehreren der Ansprüche 12 bis 23,
**dadurch gekennzeichnet,**
**dass** es oder der Transportbehälter (26) über ein Lesegerät zur Ermittlung von Informationen über Proben verfügt, welches mit einer Auswertungseinheit kommunizieren kann.

25. Membranbehältnis nach Anspruch 24,
**dadurch gekennzeichnet,**
**dass** das Lesegerät und die Auswertungseinheit Mittel für die drahtlose Kommunikation haben.

26. Membranbehältnis nach einem oder mehreren der Ansprüche 12 bis 25,
**dadurch gekennzeichnet,**
**dass** der Transportbehälter (26) eine Öffnung aufweist, welche gegen die Gasblende (16) eines Klimageräts (10) gasdicht abdichtbar ist, und dass er außerdem ein Abschlussmittel (28) aufweist, welches das Eindringen von Außenatmosphäre im Wesentlichen verhindert.

27. Membranbehältnis nach Anspruch 26,
**dadurch gekennzeichnet,**
**dass** es eine gasdichte Rückwand (29) aufweist, die gegen den Transportbehälter (26) gasdicht abschließt und die Rückwand des Transportbehälters (26) bildet.

28. Membranbehältnis nach Anspruch 27,
**dadurch gekennzeichnet,**
**dass** der Transportbehälter (26) eine Schiebevorrichtung (30) im Bereich seiner Öffnung aufweist, mit der er gasdicht abdichtbar ist, während die Öffnung gasdicht gegen die Gasblende (16) eines Klimageräts (10) abgedichtet ist.

## Claims

1. A climate control unit (10) having an inner chamber (11), which comprises a utility chamber (12), and a closable access opening (13) to the utility chamber, at least a part of the inner chamber being partitioned off and sealed in a germ-proof manner from the remainder of the inner chamber to form a germ-proof partitioned chamber (14), and the partitioning being at least partially performed by a membrane (15), which is permeable to water vapor and gas, but is impermeable to microorganisms, and it having at least one membrane container (20), which encloses at least one storage space (21) for samples in a germ-proof manner using external walls (22), at least a subarea of these external walls being formed by a membrane (15), which is permeable to water vapor and gas, but impermeable to microorganisms,
**characterized in that** the membrane container (20) has at least one charge opening (24), which is removably closable in a germ-proof manner using a closure device (25).

2. The climate control unit according to Claim 1,
**characterized in that** the membrane has pores having a pore size of at most 0.5 µm and particularly preferably less than 0.2 µm.

3. The climate control unit according to Claim 1 or 2,
**characterized in that** the climate control unit is an incubator.

4. The climate control unit according to one of the preceding claims,
**characterized in that** a gas screen (16) is situated between the access opening (13) and the interior (11) of the climate control unit, the gas screen terminating the inner chamber of the climate control unit (10).

5. The climate control unit according to one or more of Claims 1 through 4,
**characterized in that** it has a fastening device (23), on which the membrane container (20) is removably fastenable in the inner chamber (11) of the climate control unit.

6. The climate control unit according to Claim 5,
**characterized in that** the membrane container (20) is removably fastened on the gas screen (16).

7. The climate control unit according to one of the preceding claims,
**characterized in that** the closure device (25) is a door.

8. The climate control unit according to one of the preceding claims,
**characterized in that** the closure device (25) is integrated in the gas screen (16) and seals the membrane container (20) in relation to the gas screen (16).

9. The climate control unit according to one of Claims 1 through 7,
**characterized in that** the closure device (25) is fastened on the membrane container (20) and sealed in relation thereto.

10. The climate control unit according to Claim 9,
**characterized in that** the membrane container (20) is removable together with the closure device (25) from the climate control unit (10).

11. The climate control unit according to Claim 10,
**characterized in that** it has a substitute closure device, which closes a passage resulting from the removal of the membrane container (20).

12. A membrane container for use in a climate control unit according to one of Claims 10 and 11, which encloses at least one sample storage space using walls (22) in a germ-proof manner, at least a subarea of the walls being formed by a membrane (15), which is permeable to gas and water vapor, but impermeable to microorganisms, and which has at least one charge opening (24),
**characterized in that** the charge opening is removably closable in a germ-proof manner by a closure device (25), in particular by a door.

13. The membrane container according to Claim 12,
**characterized in that** the membrane has pores having a pore size of at most 0.5 µm and particularly preferably less than 0.2 µm.

14. The membrane container according to one of Claims 12 or 13,
**characterized in that** it has at least one viewing window.

15. The membrane container according to one or more of Claims 12 through 14,
**characterized in that** it has a closure device, which automatically closes it upon removal from the climate control unit.

16. The membrane container according to one or more of Claims 12 through 15,
**characterized in that** it can be folded together flat for transportation.

17. The membrane container according to one or more of Claims 12 through 16,
**characterized in that** it is manufactured from sterilizable material.

18. The membrane container according to one or more of Claims 12 through 17,
**characterized in that** it has a transport receptacle (26), in which the membrane container (20) can be enclosed tight to water vapor and gas for transport.

19. The membrane container according to Claim 18,
**characterized in that** the transport receptacle (26) is at least partially manufactured from transparent material.

20. The membrane container according to one or more of Claims 18 and 19,
**characterized in that** the transport receptacle (26) has a device for temperature control.

21. The membrane container according to one or more of Claims 18 through 20,
**characterized in that** the transport receptacle (26) has a device, using which the gas composition in the interior thereof can be influenced.

22. The membrane container according to one or more of Claims 18 through 21,
**characterized in that** the transport receptacle (26) has a humidifying device, using which the ambient humidity in the interior can be regulated.

23. The membrane container according to Claim 22,
**characterized in that** the humidifying device has at least one sponge structure impregnated with water.

24. The membrane container according to one or more of Claims 12 through 23,
**characterized in that** it or the transport receptacle (26) has a read device for ascertaining information about samples, which can communicate with an analysis unit.

25. The membrane container according to Claim 24,
**characterized in that** the read device and the analysis unit have means for wireless communication.

26. The membrane container according to one or more of Claims 12 through 25,
**characterized in that** the transport receptacle (26) has an opening, which can be sealed gas-tight in relation to the gas screen (16) of a climate control unit (10), and it additionally has closure means (28), which essentially prevent the penetration of external atmosphere.

27. The membrane container according to Claim 26,
**characterized in that** it has a gas-tight rear wall (29), which closes it gas-tight in relation to the transport receptacle (26) and forms the rear wall of the transport receptacle (26).

28. The membrane container according to Claim 27,
**characterized in that** the transport receptacle (26) has a slide device (30) in the area of its opening, using which it can be sealed gas-tight, while the opening is sealed gas-tight in relation to the gas screen (16) of a climate control unit (10).

## Revendications

1. Appareil de climatisation (10) avec un espace intérieur (11), lequel comporte un espace utile (12), et avec une ouverture d'accès (13) audit espace utile pouvant être fermée, au moins une partie dudit espace intérieur étant séparée du reste dudit espace intérieur en formant un espace (14) séparé de manière imperméable aux germes et la séparation étant au moins en partie réalisée par une membrane (15), laquelle est perméable à la vapeur d'eau et aux gaz, mais imperméable aux micro-organismes, et cette partie présentant au moins un récipient à membrane (20), qui enserre de manière étanche aux germes au moins un dépôt (21) pour des échantillons par des cloisons extérieures (22), au moins une zone partielle desdites cloisons extérieures étant formée par une membrane (15), laquelle est perméable à la vapeur d'eau et aux gaz, mais imperméable aux micro-organismes,
**caractérisé en ceci**
que ledit récipient à membrane (20) présente au moins une trappe de chargement (24), qui peut être fermée de manière amovible par un dispositif de fermeture étanche aux germes (25).

2. Appareil de climatisation selon la revendication 1,
**caractérisé en ceci**
que ladite membrane présente des pores ayant une taille d'au maximum 0,5 µm et en particulier de préférence inférieure à 0,2 µm.

3. Appareil de climatisation selon la revendication 1 ou 2,
**caractérisé en ceci**
que l'appareil de climatisation est un incubateur.

4. Appareil de climatisation selon l'une des revendications précédentes,
**caractérisé en ceci**
qu'une séparation imperméable aux gaz (16) est disposée entre ladite ouverture d'accès (13) et ledit espace intérieur (11) de l'appareil de climatisation, ladite séparation imperméable aux gaz fermant ledit espace intérieur de l'appareil de climatisation (10).

5. Appareil de climatisation selon l'une ou plusieurs des revendications 1 à 4,
**caractérisé en ceci**
qu'il présente un dispositif de fixation (23) auquel ledit récipient à membrane (20) peut être fixé de manière amovible dans ledit espace intérieur (11) de l'appareil de climatisation.

6. Appareil de climatisation selon la revendication 5,
**caractérisé en ceci**
que ledit récipient à membrane (20) est fixé de manière amovible à ladite séparation imperméable aux gaz (16).

7. Appareil de climatisation selon l'une des revendications précédentes,
**caractérisé en ceci**
que le dispositif de fermeture (25) est une porte.

8. Appareil de climatisation selon l'une des revendications précédentes,
**caractérisé en ceci**
que ledit dispositif de fermeture (25) est intégré dans ladite séparation imperméable aux gaz (16) et que ledit récipient à membrane (20) rend étanche contre ladite séparation imperméable aux gaz (16).

9. Appareil de climatisation selon l'une des revendications 1 à 7,
**caractérisé en ceci**
que ledit dispositif de fermeture (25) est fixé sur ledit récipient à membrane (20) et rend étanche par rapport à celui-ci.

10. Appareil de climatisation selon la revendication 9,
**caractérisé en ceci**
que ledit récipient à membrane (20) peut être retiré de l'appareil de climatisation (10) conjointement avec ledit dispositif de fermeture (25).

11. Appareil de climatisation selon la revendication 10,
**caractérisé en ceci**
qu'il présente un dispositif de fermeture de remplacement, lequel ferme un passage constitué par le retrait d'un récipient à membrane (20).

12. Récipient à membrane pour l'utilisation dans un appareil de climatisation selon l'une des revendications 10 et 11, lequel enserre de manière étanche aux germes au moins un dépôt (22) pour des échantillons par des cloisons, au moins une zone partielle desdites cloisons étant constituée par une membrane (15), laquelle est perméable à la vapeur d'eau et aux gaz, mais imperméable aux micro-organismes, et lequel présente au moins une trappe de chargement (24),
**caractérisé en ceci**
que ladite trappe de chargement peut être fermée de manière amovible par un dispositif de fermeture (25), en particulier par une porte étanche aux germes.

13. Récipient à membrane selon la revendication 12,
**caractérisé en ceci**
que ladite membrane présente des pores ayant une taille d'au maximum 0,5 µm et en particulier de préférence inférieure à 0,2 µm.

14. Récipient à membrane l'une des revendications 12 ou 13,
**caractérisé en ceci**
qu'il présent au moins un hublot.

15. Récipient à membrane selon l'une ou plusieurs des revendications 12 à 14,
**caractérisé en ceci**
qu'il présente un dispositif de fermeture, qui le ferme de soi-même lorsqu'on le retire de l'appareil de climatisation.

16. Récipient à membrane selon l'une ou plusieurs des revendications 12 à 15,
**caractérisé en ceci**
qu'il peut être mis à plat par pliage pour le transport.

17. Récipient à membrane selon l'une ou plusieurs des revendications 12 à 16,
**caractérisé en ceci**
qu'il est réalisé en matériel pouvant être stérilisé.

18. Récipient à membrane selon l'une ou plusieurs des revendications 12 à 17,
**caractérisé en ceci**
qu'il présente un récipient de transport (26) dans lequel ledit récipient à membrane (20) peut être enclos de manière étanche à la vapeur d'eau et aux gaz.

19. Récipient à membrane selon la revendication 18,
**caractérisé en ceci**
que ledit récipient de transport (26) est réalisé au moins en partie en un matériel transparent.

20. Récipient à membrane selon l'une ou plusieurs des revendications 18 et 19,
**caractérisé en ceci**
que le récipient de transport (26) présente un dispositif pour le tempérer.

21. Récipient à membrane selon l'une ou plusieurs des revendications 18 à 20,
**caractérisé en ceci**
que ledit récipient de transport (26) présente un dispositif par lequel la composition des gaz à l'intérieur peut être influencée.

22. Récipient à membrane selon l'une ou plusieurs des revendications 18 à 21,
**caractérisé en ceci**
que ledit récipient de transport (26) présente un dispositif d'humidification par lequel l'humidité de l'air à l'intérieur peut être régulée.

23. Récipient à membrane selon la revendication 22,
**caractérisé en ceci**
que ledit dispositif d'humidification présente au moins une structure en éponge imbibée d'eau.

24. Récipient à membrane selon l'une ou plusieurs des revendications 12 à 23,
**caractérisé en ceci**
qu'il ou ledit récipient de transport (26) dispose d'un lecteur pour l'investigation d'informations sur les échantillons, ledit lecteur pouvant communiquer avec une unité d'analyse.

25. Récipient à membrane selon la revendication 24,
**caractérisé en ceci**
que ledit lecteur et ladite unité d'analyse disposent de moyens de communication sans fil.

26. Récipient à membrane selon l'une ou plusieurs des revendications 12 à 25,
**caractérisé en ceci**
que ledit récipient de transport (26) présente une ouverture, laquelle peut être rendue étanche aux gaz par rapport à ladite séparation imperméable aux gaz (16) d'un appareil de climatisation (10), et qu'il présente de plus un moyen de fermeture (28), lequel empêche pour l'essentiel la pénétration d'atmosphère extérieure.

27. Récipient à membrane selon la revendication 26,
**caractérisé en ceci**
qu'il présente une cloison postérieure (29), laquelle ferme de manière étanche aux gaz par rapport au récipient de transport (26) et laquelle constitue la cloison postérieure dudit récipient de transport (26).

28. Récipient à membrane selon la revendication 27,
**caractérisé en ceci**
que ledit récipient de transport (26) présente un dispositif coulissant (30) au niveau de son ouverture grâce auquel il peut être fermé de manière étanche aux gaz tandis que ladite ouverture est rendue étanche par rapport à ladite séparation imperméable aux gaz (16) d'un appareil de climatisation (10).
